# EUROPEAN PATENT APPLICATION

(11) **EP 3 241 484 A1**
(43) Date of publication of application: **08.11.2017**
(21) Application number: 16869369.5
(22) Date of filing: 15.09.2016
(51) Int. Cl.: A61B 1/04, A61B 1/00, G02B 23/24

(54) **ENDOSCOPE SYSTEM AND ENDOSCOPE**

(30) Priority: 04.03.2016 JP 2016042503
(71) Applicant: Olympus Corporation, Tokyo 192-8507 (JP)
(72) Inventor: KUGIMIYA, Hideyuki, Tokyo 192-8507 (JP); KOSHIDA, Ryo, Tokyo 192-8507 (JP); YOKOUCHI, Masahito, Tokyo 192-8507 (JP)
(74) Representative: Gunzelmann, Rainer
(86) International application number: PCT/JP2016/077264
(87) International publication number: WO 2017/149814

(57) **Abstract**

An endoscope system includes an image pickup device 14 that picks up an image of an inside of a subject, and outputs two or more digital signals, an electro-optical conversion section 27 that converts the two or more digital signals outputted from the image pickup device 14 into optical signals, and outputs the optical signals, an optical transmitting section 5a that includes two or more optical transmitting members, and is adapted to transmit, in parallel, by the two or more optical transmitting members, two or more optical signals outputted from the electro-optical conversion section 27, and an output selection section 26 that is provided between the image pickup device 14 and the electro-optical conversion section 27, and is capable of combining, and outputting to one optical transmitting member, the two or more digital signals that are supplied to the two or more optical transmitting members, based on a transmission state of data optically transmitted by the optical transmitting section 5a.

## Description

### Technical Field

The present invention relates to an endoscope system and an endoscope, and more particularly, to an endoscope system and an endoscope which are capable of transmitting an image signal by an optical transmitting member.

### Background Art

Endoscopes are widely used in medical and industrial fields. An endoscope displays, at a display device, an image of an object acquired through an observation window provided at a distal end of an insertion section as an endoscopic image, and is used for examination and the like.

An object image is photoelectrically converted by an image pickup device, and an image pickup signal is outputted as an image signal from the endoscope through a wire.

Furthermore, in recent years, due to an increased number of pixels in an image pickup device, a technique has been proposed which transmits an image signal by an optical fiber, which is an optical transmitting member.

An optical fiber is susceptible to bending stress and is easily damaged or disconnected, and thus, International Publication No. WO2012/046856 proposes and discloses an endoscope system which includes two transmitting means for transmitting an image signal as an optical signal and an electrical signal and which is capable of switching between and outputting the optical signal and the electrical signal so that observation of an object by the endoscope can be continued even when an optical fiber is damaged, for example.

The proposed endoscope system includes an endoscope and a signal processing device, and the signal processing device is configured to select one of pixel information by an optical signal and pixel information by an electrical signal according to presence/absence of a transmission abnormality for the optical signal, such as presence/absence of disconnection of an optical fiber.

However, when the amount of data of an image signal is increased due to an increased number of pixels in an image pickup device, if transmission by an electrical signal is selected, because the transmission speed of an image signal which is transmitted by an electrical signal is slow, an appropriate image may not be generated.

Accordingly, the present invention has its object to provide an endoscope system and an endoscope which are capable of appropriately transmitting an image signal even when the amount of data of the image signal is large and when an abnormality is present in the transmission by an optical signal.

### Disclosure of Invention

### Means for Solving the Problem

An endoscope system according to an aspect of the present invention includes an image pickup section that picks up an image of an inside of a subject, and outputs two or more digital signals, an electro-optical conversion section that converts the two or more digital signals outputted from the image pickup section into optical signals, and outputs the optical signals, an optical transmitting section that includes two or more optical transmitting members, and is adapted to transmit, in parallel, by the two or more optical transmitting members, two or more optical signals outputted from the electro-optical conversion section, and a signal output section that is provided between the image pickup section and the electro-optical conversion section, and is capable of combining, and outputting to one optical transmitting member, the two or more digital signals that are supplied to the two or more optical transmitting members, based on a transmission state of data optically transmitted by the optical transmitting section.

An endoscope according to an aspect of the present invention includes an image pickup section that picks up an image of an inside of a subject, and outputs two or more digital signals, an electro-optical conversion section that includes two or more electro-optical converters, and converts the two or more digital signals outputted from the image pickup section into optical signals and outputs the optical signals in parallel from the two or more electro-optical converters, and a signal output section that is provided between the image pickup section and the electro-optical conversion section, and is capable of combining, and outputting to one electro-optical converter, the two or more digital signals that are supplied to the two or more electro-optical converters.

### Brief Description of the Drawings

Fig. 1 is a configuration diagram of an endoscope system according to an embodiment of the present invention;
Fig. 2 is a diagram for describing division of an endoscopic image according to the embodiment of the present invention;
Fig. 3 is a block diagram showing a configuration of an endoscope system 1 according to the embodiment of the present invention;
Fig. 4 is a block diagram showing a configuration of an output selection section 26 according to the embodiment of the present invention;
Fig. 5 is a diagram showing a connection state of a switch section 53 according to the embodiment of the present invention; and
Fig. 6 is a diagram showing output timings of signals from four output ends Oa to Od according to the embodiment of the present invention.

### Best Mode for Carrying Out the Invention

Hereinafter, an embodiment of the present invention will be described with reference to the drawings.

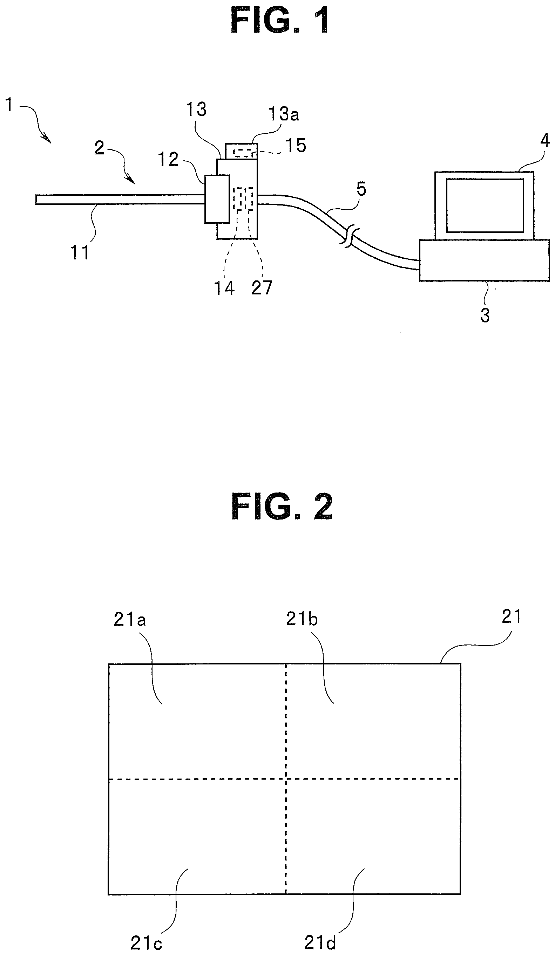

Fig. 1 is a configuration diagram of an endoscope system according to the present embodiment. An endoscope system 1 is configured by including an endoscope 2, a video processor 3, and a display device 4. The endoscope 2 and the video processor 3 are connected by a universal cable 5.

The endoscope 2 is a rigid endoscope, and includes an insertion section 11, an eyepiece section 12, and a camera head 13.

The insertion section 11 includes an objective lens system and a relay lens system which are disposed inside a rigid tubular member. The eyepiece section 12 is provided to a proximal end portion of the insertion section 11. The eyepiece section 12 includes an eyepiece lens, and a surgeon can look at an image of an object by bringing an eye close to the eyepiece section 12.

Moreover, the endoscope 2 includes the camera head 13 which is detachably mounted to the eyepiece section 12.

An image pickup device 14 is built in the camera head 13. The image pickup device 14 includes an image pickup surface which receives light which has passed through the eyepiece lens of the eyepiece section 12. In the present case, the image pickup device 14 outputs an image signal of 4K resolution or 8K resolution, for example.

Furthermore, the camera head 13 is provided with an operation section 13a including various switches, such as a freeze button, which can be operated by a user.

The video processor 3 is a signal processing device which performs various types of image processing on an image signal received from the endoscope 2, and which outputs the result to the display device 4. An endoscopic image is displayed on the display device 4.

The universal cable 5 includes a plurality (in the present case, four) of optical fibers, which are optical transmitting members, and image signals containing pixel information obtained by the image pickup device 14 are transmitted from the camera head 13 to the video processor 3 by an optical signal through the plurality of optical fibers.

Note that, in the present case, the eyepiece section 12 and the camera head 13 are separate from each other, but the eyepiece section 12 and the camera head 13 may alternatively be integrated in an inseparable manner.

Note that the plurality of optical fibers disposed in the universal cable 5 are made of quartz glass and are formed to have an extremely small diameter (such as 0.125 mm), and are thus extremely fragile, and each optical fiber is protected by being primarily coated with ultraviolet curing resin, for example, and then being covered with a protective tube. The plurality of primarily coated optical fibers may be covered by one protective tube, or each of the plurality of primarily coated optical fibers may be individually covered by a protective tube.

To increase the number of pixels, an image of one frame of an endoscopic image is divided into a plurality of pixel regions, and the endoscope 2 transmits an image signal to the video processor 3 through the universal cable 5 on a per pixel region basis.

Fig. 2 is a diagram for describing division of an endoscopic image. Fig. 2 shows a case where an endoscopic image 21X is divided into a plurality of pieces, or in the present case, four.

More specifically, the endoscopic image 21X of one frame is divided into four pixel regions 21a, 21b, 21c, and 21d. Respective image signals of the pixel regions each contain a plurality of pieces of pixel information of the pixel region, and the signals are transmitted to the video processor 3 in parallel.

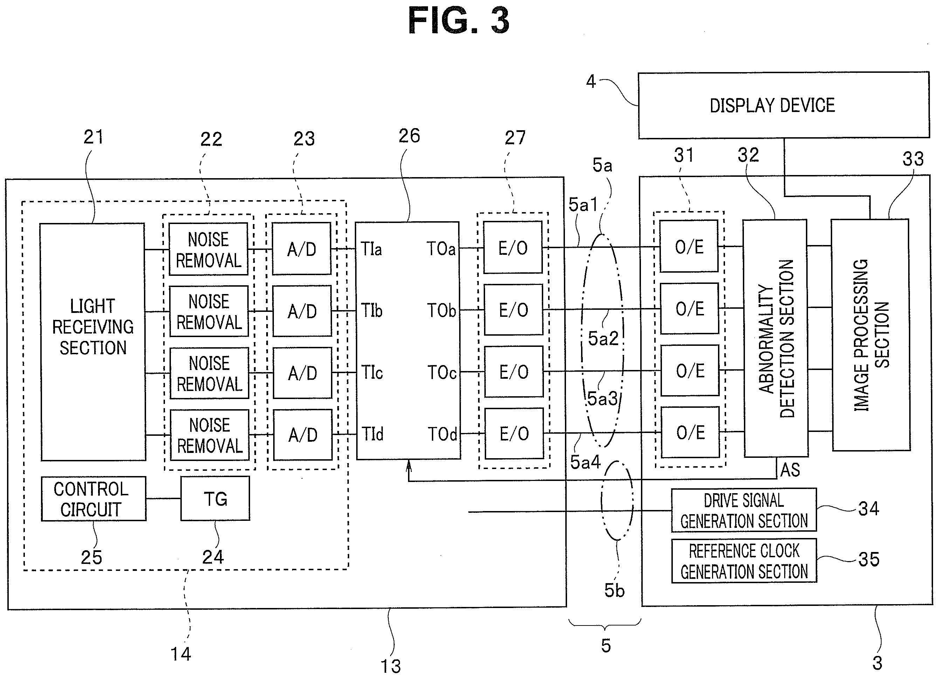

Fig. 3 is a block diagram showing a configuration of the endoscope system 1.

The universal cable 5 is connected to the camera head 13, and the universal cable 5 extending from the camera head 13 is connected to the video processor 3 by a connector, not shown. A plurality of optical fibers 5a and a plurality of electrical wires 5b are inserted in the universal cable 5. The plurality of wires 5b include a metal transmitting member, such as a metal conductive wire, and as described below, the wires 5b configure a metal transmitting section which transmits information about an abnormality to an output selection section 26 when an abnormality is detected by an abnormality detection section 32.

The image pickup device 14 in the camera head 13 is a CMOS image sensor, and includes a light receiving section 21 including a light receiving surface, a noise removal section 22, an analog-digital conversion section (hereinafter abbreviated as A/D) 23, a timing generator (hereinafter abbreviated as TG) 24, and a control circuit 25.

The image pickup device 14 is a CMOS image pickup device, and the light receiving surface of the light receiving section 21 is divided into four pixel regions 21 a, 21 b, 21 c, and 21 d. An image signal photoelectrically converted by the light receiving section 21 is outputted to the noise removal section 22.

More specifically, image signals of the four pixel regions 21a, 21b, 21 c, and 21d are outputted to four respective noise removal circuits in the noise removal section 22. The noise removal section 22 outputs, to the A/D 23, respective image signals of the pixel regions from which noise has been removed.

The image pickup device 14 thus configures an image pickup section which picks up an image of the inside of a subject and which outputs two or more digital signals, or in the present case, digital signals of four pixel regions. The two or more digital signals outputted from the image pickup device 14 correspond to two or more image pickup areas obtained by dividing an image obtained by picking up an image of an object.

The A/D 23 includes analog-digital conversion circuits for respective pixel regions, and converts image signals from analog signals to digital signals and outputs the signals to the output selection section 26.

The TG 24 generates various timing signals, and outputs the signals to the control circuit 25. The control circuit 25 drives the light receiving section 21, the noise removal section 22, and the A/D 23 based on the various timing signals.

The output selection section 26 is provided inside the camera head 13, and includes four input terminals TIa, TIb, TIc, and TId, and four output terminals TOa, TOb, TOc, and TOd. The four input terminals TIa, TIb, TIc, and TId correspond to the four pixel regions 21a, 21b, 21c, and 21d, respectively. Four image signals of the four pixel regions 21a, 21b, 21c, and 21d are inputted to the four input terminals TIa, TIb, TIc, and TId, respectively. The output selection section 26 is connected to the electro-optical conversion section 27.

The output selection section 26 is a circuit which selects from which of the four output terminals TOa, TOb, TOc, and TOd four image signals inputted to the four input terminals TIa, TIb, TIc, and TId are to be outputted, and outputs the signals to the electro-optical conversion section 27.

Note that, in the present case, the output selection section 26 is provided inside the camera head 13 as a different circuit from the image pickup device 14, but the output selection section 26 may be mounted on a circuit board 15 provided in the operation section 13a, or may be provided in a chip of the image pickup device 14, which is a CMOS image sensor, for example.

The configuration of the output selection section 26 will be described below.

The electro-optical conversion section 27 is provided inside the camera head 13. The electro-optical conversion section 27 includes four electro-optical converters (E/O) corresponding, respectively, to the four output terminals TOa, TOb, TOc, and TOd of the output selection section 26. The four electro-optical converters are connected, respectively, to four optical fibers 5a1 to 5a4, which are inserted in the universal cable 5.

That is, the electro-optical conversion section 27 includes two or more electro-optical converters, and converts two or more digital signals outputted from the image pickup device 14 into optical signals and outputs the optical signals in parallel from the two or more electro-optical converters. A plurality of optical fibers 5a configure an optical transmitting section which includes two or more optical transmitting members, and which is adapted to transmit, in parallel, by the two or more optical transmitting members, two or more optical signals outputted from the electro-optical conversion section 27.

The video processor 3 includes a photoelectric conversion section 31, an abnormality detection section 32, an image processing section 33, a drive signal generation section 34, and a reference clock generation section 35.

The photoelectric conversion section 31 includes four photoelectric conversion circuits (O/E) corresponding to the four optical fibers 5a1 to 5a4. That is, the photoelectric conversion section 31 converts optical signals transmitted by a plurality of optical fibers 5a, which are the optical transmitting section, into electrical signals.

The photoelectric conversion section 31 is connected to the abnormality detection section 32. The abnormality detection section 32 is a circuit which monitors an output of each photoelectric conversion circuit (O/E), and which detects an abnormality by determining whether an abnormality is present in four optical signals. An abnormality refers to an instance where an optical signal is missing, or a noise level is at a predetermined value or higher, for example.

That is, electrical signals outputted by the photoelectric conversion section 31 are inputted to the abnormality detection section 32, and the abnormality detection section 32 detects an abnormality in the plurality of optical fibers 5a based on the electrical signals.

When an abnormality is detected, the abnormality detection section 32 generates and outputs a predetermined abnormality detection signal AS. The abnormality detection signal AS is supplied to the output selection section 26 through one of the plurality of wires 5b inserted in the universal cable 5. The abnormality detection signal AS contains information indicating the optical fiber where the abnormality is detected. That is, in the case where an abnormality is detected, the abnormality detection section 32 transfers to the output selection section 26, which is a signal output section, information about the optical fiber where the abnormality is present.

Also, the abnormality detection section 32 transmits a received image signal of each pixel region to the image processing section 33.

The image processing section 33 combines image signals received via the abnormality detection section 32, performs predetermined image processing, and generates an endoscopic image. The image signal of the generated endoscopic image is supplied to the display device 4, and the endoscopic image is displayed on a display screen.

The drive signal generation section 34 generates drive signals for driving various circuits in the image pickup device 14, and supplies the signals to the camera head 13 through one or some of the plurality of wires 5b.

The reference clock generation section 35 generates a reference clock as the reference timing for driving various circuits in the video processor 3.

Note that, in the present case, the drive signal generation section 34 is provided inside the video processor 3, but the drive signal generation section 34 may alternatively be provided inside the camera head 13.

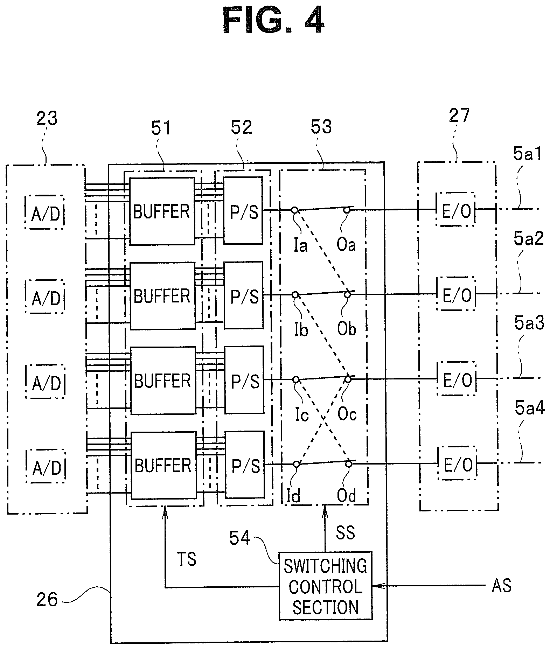

Fig. 4 is a block diagram showing a configuration of the output selection section 26.

The output selection section 26 is configured by including a buffer section 51, a parallel-serial conversion section 52, a switch section 53, and a switching control section 54.

The buffer section 51 includes four buffer circuits corresponding to the four input terminals TIa, TIb, TIc, and TId, and each buffer circuit stores an image signal from the corresponding analog-digital conversion circuit. Also, each buffer circuit includes a function of delaying an output timing of a stored image signal based on a delay instruction signal TS from the switching control section 54.

The parallel-serial conversion section 52 includes four parallel-serial conversion circuits, and an image signal from the corresponding buffer circuit is inputted to each parallel-serial conversion circuit, and the parallel-serial conversion circuit converts the signal into a serial signal and outputs the signal.

Note that, in the present case, the buffer section 51 is provided on the input side of the parallel-serial conversion section 52, but the buffer section 51 may alternatively be provided on the output side of the parallel-serial conversion section 52.

The switch section 53 is a circuit which switches the connection state between the four input ends Ia to Id and the four output ends Oa to Od. Outputs of the four parallel-serial conversion circuits of the parallel-serial conversion section 52 are connected to the four respective input ends Ia to Id.

The switch section 53 is a circuit which switches the connection state between the four input ends Ia to Id and the four output ends Oa to Od based on a switching instruction signal SS from the switching control section 54.

Normally, that is, when an abnormality is not detected by the abnormality detection section 32 regarding optical transmission, output destinations of the four input ends Ia to Id are selected by the switch section 53 so that the four input ends Ia to Id are connected to the four output ends Oa to Od, respectively, as shown by solid lines in Fig. 4.

Furthermore, when the abnormality detection section 32 detects an abnormality regarding optical transmission, the switch section 53 is capable of performing switching so that the input end Ia is connected to the output end Ob, the input end Ib is connected to the output end Oc, the input end Ic is connected to the output end Od, and the input end Id is connected to the output end Oc, as shown by dotted lines in Fig. 4.

That is, the output selection section 26 configures a signal output section which is provided between the image pickup device 14 and the electro-optical conversion section 27, and which is capable of combining two or more digital signals that are supplied to two or more optical transmitting members and of outputting the result to one optical transmitting member, based on the transmission state of data optically transmitted by a plurality of optical fibers 5a.

Note that the connection relationship between each input end and each output end at the time of abnormality detection shown by the dotted lines in Fig. 4 is only an example, and connection relationships other than the connection relationship shown by the dotted lines in Fig. 4 are also applicable.

The switching control section 54 switches the connection state between the four input ends Ia to Id and the four output ends Oa to Od based on an abnormality detection signal AS from the abnormality detection section 32 in such a way that an image signal which was being transmitted by an optical fiber where an abnormality is detected is combined with an image signal of another pixel region and is transmitted by an optical fiber where an abnormality is not detected.

The output selection section 26, which is the signal output section, combines a digital signal corresponding to an optical transmitting member where an abnormality is present and a digital signal corresponding to another optical transmitting member and output combined signals so that transmission by the other optical transmitting member is enabled.

The manner of transmission, by using another optical fiber, at the time of detection of an abnormality in an optical fiber, of an image signal which was being transmitted by the optical fiber where the abnormality is detected is determined in advance, according to the abnormal state, for the switching control section 54.

In the present case, when an abnormality is detected in the optical fiber 5a1, the input end Ia is connected to the output end Ob. When an abnormality is detected in the optical fiber 5a2, the input end Ib is connected to the output end Oc. When an abnormality is detected in the optical fiber 5a3, the input end Ic is connected to the output end Od. When an abnormality is detected in the optical fiber 5a4, the input end Id is connected to the output end Oc.

The switching control section 54 switches, based on an abnormality detection signal AS from the abnormality detection section 32, the connection state between the four input ends Ia to Id and the four output ends Oa to Od at the switch section 53 to a connection state that is determined in advance, and indicates the output timing of each of two or more combined image signals.

The switching control section 54 outputs a switching instruction signal SS determined in advance to the switch section 53 and outputs a delay instruction signal TS determined in advance to the buffer section 51 according to an expected abnormality in an optical fiber.

The switching control section 54 may be realized by a central processing unit (CPU) and a memory, or by a logic circuit.

For example, when the optical fiber 5a1 is disconnected, and the abnormality is detected by the abnormality detection section 32, an abnormality detection signal AS is outputted to the switching control section 54. The abnormality detection signal AS contains information indicating that an abnormality is present in the optical fiber 5a1.

The switching control section 54 is configured in such a manner that, when an abnormality is in the optical fiber 5a1, a switching instruction signal SS and a delay instruction signal TS are outputted to the switch section 53 and the buffer section 51, respectively, so that an image signal of the pixel region 21a which was being transmitted by the optical fiber 5a1 is added to an image signal of the pixel region 21b originally transmitted by the optical fiber 5a2 so as to be transmitted by the optical fiber 5a2.

The abnormality detection signal AS contains information indicating the optical fiber where an abnormality is present, and thus, the switching instruction signal SS is a signal indicating that a connection state according to the abnormality detection signal AS is to be reached, and the delay instruction signal TS is a signal indicating that an image signal is to be outputted from each buffer circuit at an output timing according to the abnormality detection signal AS.

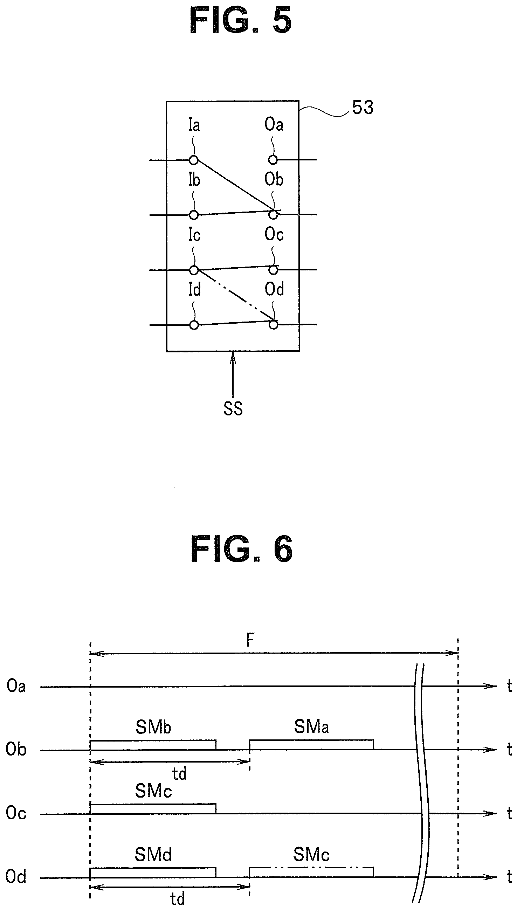

Fig. 5 is a diagram showing a connection state of the switch section 53. Fig. 6 is a diagram showing output timings of signals from the four output ends Oa to Od.

Fig. 5 shows a case where an abnormality is detected in the optical fiber 5a1, and an image signal SMa of the pixel region 21a and an image signal SMb of the pixel region 21b are transmitted by using the optical fiber 5a2. As shown in Fig. 5, when an abnormality detection signal AS containing information indicating that an abnormality is present in the optical fiber 5a1 is received, the switching control section 54 outputs, to the switch section 53, a switching instruction signal SS for changing the connection state of the internal switch so as to connect the input end Ia and the input end Ib to the output end Ob.

Furthermore, when the abnormality detection signal AS containing information indicating that an abnormality is present in the optical fiber 5a1 is received, the switching control section 54 outputs, to the buffer section 51, a delay instruction signal TS for delaying the output timing of the image signal SMa of the pixel region 21a from the buffer circuit by a predetermined period of time td.

In the case of Fig. 6, the delay instruction signal TS for delaying the output timing of a signal by the period of time td is outputted from the switching control section 54 for the buffer circuit corresponding to the input end Ia.

The example described above refers to a case where one optical fiber is disconnected, but it is also possible that two optical fibers become disconnected.

For example, when the optical fibers 5a1 and 5a3 are disconnected, the internal connection state of the switch section 53 is changed such that the input end Ic and the input end Id are connected to the output end Od, as shown by a two-dot chain line in Fig. 5.

Moreover, as shown by a two-dot chain line in Fig. 6, the delay instruction signal TS for delaying output by the period of time td is outputted from the switching control section 54 to the buffer circuit corresponding to the input end Ic.

Furthermore, although not shown, when the optical fibers 5a2 and 5a4 are disconnected, the connection state of an internal switch at the switch section 53 is changed so that the input ends Ib, Ic, and Id are connected to the output end Od, and a delay instruction signal TS for delaying output from the buffer circuit corresponding to the input end Ib by a period of time td and for delaying output from the buffer circuit corresponding to the input end Id by a period of time 2td is outputted from the switching control section 54.

As described above, according to the endoscope system 1, an image signal is divided and the obtained signals are transmitted in parallel by a plurality of optical fibers from the endoscope 2 to the video processor 3, and when an abnormality in optical transmission is detected, an image signal of a pixel region which was being transmitted by an optical fiber where the abnormality is detected is transmitted by a normal optical fiber by being combined with an image signal of another pixel region.

Accordingly, according to the embodiment described above, an endoscope system and an endoscope which are capable of appropriately transmitting an image signal even when the amount of data of the image signal is large and when an abnormality is present in the transmission by an optical signal may be provided.

Note that, in the embodiment described above, the endoscope is a rigid endoscope, but the endoscope may alternatively be a flexible endoscope, an insertion section of which has flexibility.

The present invention is not limited to the embodiment described above, and various changes and modifications may be made within the scope of the present invention.

The present application is based upon and claims priority from Japanese Patent Application No. 2016-42503 filed in Japan on March 4, 2016, the disclosed contents of which are incorporated in the present specification and claims by reference.

## Claims

1. An endoscope system comprising:
an image pickup section that picks up an image of an inside of a subject, and outputs two or more digital signals;
an electro-optical conversion section that converts the two or more digital signals outputted from the image pickup section into optical signals, and outputs the optical signals;
an optical transmitting section that includes two or more optical transmitting members, and is adapted to transmit, in parallel, by the two or more optical transmitting members, two or more optical signals outputted from the electro-optical conversion section; and
a signal output section that is provided between the image pickup section and the electro-optical conversion section, and is capable of combining, and outputting to one optical transmitting member, the two or more digital signals that are supplied to the two or more optical transmitting members, based on a transmission state of data optically transmitted by the optical transmitting section.

2. The endoscope system according to claim 1, further comprising a photoelectric conversion section that converts an optical signal transmitted by the optical transmitting section into an electrical signal.

3. The endoscope system according to claim 2, further comprising an abnormality detection section to which the electrical signal outputted by the photoelectric conversion section is inputted, and that detects an abnormality in the optical transmitting section based on the electrical signal.

4. The endoscope system according to claim 3, wherein
the abnormality detection section transmits to the signal output section, when the abnormality is detected, information about an optical transmitting member where the abnormality is present, and
the signal output section combines a digital signal corresponding to the optical transmitting member where the abnormality is present and a digital signal corresponding to another optical transmitting member and output combined signals so that transmission by the other optical transmitting member is enabled.

5. The endoscope system according to claim 3, further comprising a metal transmitting section that includes a metal transmitting member, and transmits, when an abnormality is detected by the abnormality detection section, information about the abnormality to the signal output section.

6. The endoscope system according to claim 1, wherein the two or more digital signals outputted from the image pickup section correspond to two or more image pickup areas obtained by dividing an image obtained by picking up an image of the object.

7. The endoscope system according to claim 1, wherein the signal output section is provided inside a camera head as a different circuit from the image pickup device.

8. The endoscope system according to claim 1, wherein
the image pickup device is configured by a CMOS image sensor, and
the signal output section is formed in a chip of the image pickup device.

9. An endoscope comprising:
an image pickup section that picks up an image of an inside of a subject, and outputs two or more digital signals;
an electro-optical conversion section that includes two or more electro-optical converters, and converts the two or more digital signals outputted from the image pickup section into optical signals and outputs the optical signals in parallel from the two or more electro-optical converters; and
a signal output section that is provided between the image pickup section and the electro-optical conversion section, and is capable of combining, and outputting to one electro-optical converter, the two or more digital signals that are supplied to the two or more electro-optical converters.
